(19) Europäisches Patentamt
European Patent Office
Office européen des brevets

(11) **EP 2 853 196 B1**

(12) **EUROPÄISCHE PATENTSCHRIFT**

(45) Veröffentlichungstag und Bekanntmachung des Hinweises auf die Patenterteilung:
**11.05.2016 Patentblatt 2016/19**

(51) Int Cl.:
*A61B 5/053* *(2006.01)*      *A61B 5/085* *(2006.01)*

(21) Anmeldenummer: **13186489.4**

(22) Anmeldetag: **27.09.2013**

(54) **Elektroimpedanztomographie-Gerät und -Verfahren**

Electro-impedance tomography apparatus and method

Appareil et procédé de tomographie par impédance électrique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.04.2015 Patentblatt 2015/14**

(73) Patentinhaber: **Drägerwerk AG & Co. KGaA 23558 Lübeck (DE)**

(72) Erfinder: **Gärber, Yvo 23881 Breitenfelde (DE)**

(56) Entgegenhaltungen:
**EP-A1- 1 000 580      US-A1- 2002 193 700**

- **GOMEZ-LABERGE C ET AL: "A Unified Approach for EIT Imaging of Regional Overdistension and Atelectasis in Acute Lung Injury", IEEE TRANSACTIONS ON MEDICAL IMAGING, IEEE SERVICE CENTER, PISCATAWAY, NJ, US, Bd. 31, Nr. 3, 2. März 2012 (2012-03-02), Seiten 834-842, XP011491067, ISSN: 0278-0062, DOI: 10.1109/TMI.2012.2183641**
- **FRERICHS I ET AL: "Regional respiratory inflation and deflation pressure volume curves determined by electrical impedance tomography;Regional respiratory inflation and deflation pressure--volume curves determined by electrical impedance tomography", PHYSIOLOGICAL MEASUREMENT, INSTITUTE OF PHYSICS PUBLISHING, BRISTOL, GB, Bd. 34, Nr. 6, 29. Mai 2013 (2013-05-29), Seiten 567-577, XP020245474, ISSN: 0967-3334, DOI: 10.1088/0967-3334/34/6/567**

**Beschreibung**

Die vorliegende Erfindung betrifft ein Elektroimpedanztomographie-Gerät mit: einer Mehrzahl von um den Thorax eines Patienten anbringbaren, eine Elektrodenebene aufspannenden Elektroden, einer Anzeigeeinrichtung und einer Steuer- und Auswerteeinheit, die mit den Elektroden verbunden ist und durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare jeweils ein Spannungssignal oder Stromsignal als Messsignal aufzunehmen und sukzessive andere Elektrodenpaare als einspeisende Elektrodenpaare fungieren zu lassen, um aus den Messsignalen mit einem Rekonstruktionsalgorithmus eine Matrix aus Bildelementen zu rekonstruieren, die eine räumliche Verteilung der Impedanzänderungen in der Elektrodenebene repräsentiert, und über wenigstens eine Inspiration und mindestens eine Exspiration, die aufeinander folgen, wiederholt Messsignale aufzunehmen und Matrizen zu rekonstruieren, um eine Zeitreihe von Matrizen mit Impedanzänderungen der Bildelemente über die mindestens ein Inspiration und die mindestens eine Exspiration zu erhalten.

Ein derartiges Elektroimpedanztomographie-Gerät (EIT-Gerät) ist zum Beispiel aus EP 1 000 580 A1 bekannt, das zur Aufnahme eines "Elektroimpedanztomographiebildes" eines Thoraxquerschnitts eines Patienten dient.

Auch ist ein Elektroimpedanztomographie-Gerät und -Verfahren z.B. bekannt aus GOMEZ-LABERGE C ET AL: "A Unified Approach for EIT Imaging of Regional Overdistension and Atelectasis in Acute Lung Injury", IEEE TRANSACTIONS ON MEDICAL IMAGING, IEEE SERVICE CENTER, PISCATAWAY, NJ, US, Bd. 31, Nr. 3, März 2012, Seiten 834-842.

Die elektrische Impedanztomographie ist ein Verfahren zur Rekonstruktion von Impedanzverteilungen, genauer gesagt von Impedanzänderungen bezüglich einer Referenzverteilung, in elektrisch leitfähigen Körpern. Hierzu wird an der Oberfläche des zu untersuchenden Körpers eine Vielzahl von Elektroden angebracht. In typischen Fällen wird eine ringförmige äquidistante Anordnung von 16 Elektroden verwendet, die mit einem Gurt um den Thorax eines Patienten gelegt werden können. Die Steuer- und Auswerteeinheit verfügt auch über analoge elektrische Schaltungen für die Signalverstärkung und für die Wechselstromeinspeisung und über elektronische Schaltungen zur Digitalisierung und Vorverarbeitung der Spannungssignale und über einen digitalen Signalprozessor zum Steuern des Gerätes und zur Verarbeitung der aufgenommenen Daten zur Rekonstruktion der Impedanzverteilung. Die Steuer- und Auswerteeinheit sorgt dafür, dass aufeinanderfolgend jeweils ein Paar (vorzugsweise) benachbarter Elektroden mit einem elektrischen Wechselstrom versorgt wird (z.B. 5 mA bei 50 kHz) und die elektrischen Spannungen an mehreren verbleibenden Elektrodenpaaren von der Steuer- und Auswerteeinheit erfasst werden (grundsätzlich kann auch umgekehrt eine Wechselspannung an ein Elektrodenpaar eingespeist und die Wechselströme über mehrere verbleibende Elektrodenpaare gemessen werden); typischerweise werden die Spannungen aller verbleibenden Paare von benachbarten Elektroden erfasst, grundsätzlich ist es aber auch möglich, einzelne Elektroden auszulassen, wodurch allerdings Informationen verloren gehen. Aus der Gesamtheit aller Messsignale bei den aufeinanderfolgenden Stromeinspeisungen, bei denen die Lage des einspeisenden Elektrodenpaars Schritt für Schritt um den Elektrodenring wandert, kann mit Algorithmen die Impedanzverteilung, genauer gesagt deren Änderung der Impedanz gegenüber einer Referenzverteilung (z.B. die Impedanzverteilung bei der ersten Aufnahme), rekonstruiert werden. Die bekannten Algorithmen liefern als Rekonstruktionsergebnis eine Matrix aus 32x32 Bildelementen, wobei die Matrix für jedes Bildelement die rekonstruierte Impedanzänderung für dieses Bildelement enthält. Es wird während mindestens einer Inspiration und mindestens einer Exspiration; z.B. über einen Atemzug mit Inspiration und darauf folgender Exspiration eine Vielzahl von solchen Matrizen in vorgegebenen zeitlichen Abständen aufgenommen. Diese werden aufeinanderfolgend auf einer Anzeige zur Anzeige gebracht, wodurch der intratidale zeitliche Verlauf der Impedanzverteilung praktisch als Film sichtbar gemacht wird.

Die Thorax-Elektroimpedanztomographie zur Messung der regionalen Lungenventilation findet zunehmende Verbreitung in der forschungsinteressierten Intensivmedizin. Theoretische Modelle und experimentelle Vergleiche von EIT- mit CT-Aufnahmen des Thorax zeigen eine nahezu vollständige Proportionalität von Luftgehalt des Lungengewebes und dessen Impedanz. Die Atemzüge werden räumlich mit etwa 20% des Thoraxdurchmessers und zeitlich typischerweise mit etwa 20 bis etwa 40 Matrizen pro Sekunde aufgelöst, was ein bettseitiges Monitoring der regionalen Lungenventilation ermöglicht. Die Matrizen werden gelegentlich auch als Bilder der Impedanzverteilung (mit 32x32 = 1024 Bildelementen) oder als Frames bezeichnet.

Es wird also für jedes Bildelement über eine Inspirations- oder Exspirationsphase eine Folge von Impedanzänderungen bestimmt, die hier auch als Zeitreihe von Impedanzänderungswerten für dieses Bildelement bezeichnet wird. Im Folgenden werden die Begriffe Zeitreihen von Impedanzänderungswerten und Impedanzänderungskurven mit gleicher Bedeutung verwendet, obwohl eine Zeitreihe aus diskreten Punkten im strengen Sinne keine Kurve ist. Auch in den Darstellungen werden die Zeitreihen aus Darstellungsgründen in Kurvenform als Funktionen der Zeit dargestellt.

Ein wesentlicher Vorteil der hohen Frame-Rate besteht darin, dass die Atemzüge, insbesondere deren Inspirations- und Exspirationsphase, zeitlich aufgelöst werden können. Es ist daher nicht nur möglich, die regionale Verteilung der ventilierten Luft am end-inspiratorischen Zustand (Tidal-Bild) zu analysieren, sondern auch das zeitliche Verhalten während der Inspiration und Exspiration zu untersuchen, um daraus Rückschlüsse über regionale lungenmechanische Abläufe zu gewinnen. So wird zum Beispiel in dem Artikel "Neue Verfahren zur Verbesserung der Abbildungsqualität bei funktionellen EIT-Tomogrammen der Lunge", G. Kühnel et. al., Biomed. Tech. (Berl.) 42 (1997) Suppl. 470-1 das Verhalten der lokalen Impedanzänderungskurven gegenüber der globalen Impedanzänderungskurve betrachtet, jedoch wurde das unterschiedliche Verhalten während der Inspiration und der Exspiration nicht berücksichtigt, sondern aus dem Anstieg einer angepassten Geraden eine "Filling-Capacity" bestimmt, was eine dem Tidal-Bild verwandte Größe darstellt (das *müsste etwas genauer erklärt werden*). In dem Artikel "Regional ventilation delay index: detection of tidal recruitment using electric impedance tomography", T. Muders et al, Vincent JL, editor, Yearbook of Intensive Care and Emergency Medicine, werden lokale Inspirationskurven bestimmt und für jede lokale Inspirationskurve jeweils die Zeit, zu der sie 40% ihres Maximalwertes erreicht hat, ins Verhältnis zur globalen Inspirationszeit gesetzt und daraus ein Bild von schnelleren oder langsameren Regionen mit kleineren oder größeren Zeitkonstanten als im Durchschnitt generiert. Daraus wird ein "Regional ventilation delay-index (RVD)" als Maß für die zeitliche Inhomogenität definiert.

Keines der Verfahren berücksichtigt jedoch gleichzeitig die Inspirations- und die Exspirationskurve und somit, wie viel Atemarbeit auf alveolarer Ebene dissipiert wird. Im Idealfall wird die Atemarbeit auf alveolarer Ebene nahezu vollständig während der Einatmung elastisch durch Ausdehnung der Alveolen und des Thorax gespeichert und die gespeicherte Energie während der Ausatmung passiv wieder freigegeben. In diesem Fall liegt keine Hysterese zwischen Inspiration und Exspiration vor. Dies gilt natürlich nur auf alveolarer Ebene. Wenn man den normalen Atemdruck am Mundstück oder an den oberen Bronchien misst, erhält man eine Hysterese bereits aufgrund der volumenabhängigen Reibungsverluste des Luftstroms in den Atemwegen. Diese können durch Datenanpassung an Bewegungsgleichungen atemmechanischer Modelle abgeschätzt und herausgerechnet werden.

Ein einfaches atemmechanisches Modell bei künstlicher Beatmung, bei dem die Lunge als ein Kompartiment mit Reibungswiderstand des Tubusschlauches und der Atemwege R und der Systemcompliance C unter Vernachlässigung von Trägheits- und Turbulenzverlusten sowie des Zwerchfelldrucks betrachtet wird, führt auf die Bewegungsgleichung:

$$p_0(t) = R(V)\frac{dV}{dt} + \frac{V(t)}{C(p)} = p_R(t) + p_{alv}(t)$$

Darin ist V(t) das respirierte Luftvolumen und $p_0(t)$ der obere Atemwegsdruck. Der erste Term $p_R(t)$ stellt den reibungsbedingten Druckabfall an den Atemwegen dar, während der zweite Term $p_{alv}(t)$ als mittlerer Alveolardruck interpretiert werden kann (mittlerer Alveolardruck bedeutet hier gemittelt über alle Alveolen, wobei dieser mittlere Alveolardruck dann über den Atemzug eine Funktion der Zeit ist).

Bei gesunder Lunge kann bei geringen Druckunterschieden zwischen Inspiration und Exspiration die Compliance C als konstant, also druckunabhängig angenommen werden. In diesem Modell ist somit das Volumen linear abhängig vom mittleren Alveolardruck $p_{alv}(t)$, der wiederum proportional zur globalen Impedanzänderung ist (als globale Impedanzänderung wird hier die Summe über alle lokalen Impedanzänderungen der einzelnen Bildelemente bezeichnet). Es sollte daher keine Hysterese zwischen Alveolardruck $p_{alv}(t)$ und Volumenkurve V(t) und somit zur globalen Impedanzänderungskurve $Z_{glo}(t)$ vorliegen, (wohl aber im Allgemeinen zwischen Volumen V(t) und Atemwegsdruck $p_0(t)$).

Im Falle einer räumlich und zeitlich homogenen Lunge sollte das auch für die lokalen Impedanzänderungskurven (d.h. die Impedanzänderung in einem Bildpunkt in der Bildebene) gelten, d.h. es sollte keine Hysterese zwischen dem mittleren Alveolardruck $p_{alv}(t)$ und den lokalen Impedanzänderungskurven geben. Wenn aber Teile der Alveolen nicht mehr im rein elastischen Bereich arbeiten, entsteht dort eine alveolare p-V-Hysterese.

Es ist Aufgabe der vorliegenden Erfindung, ein Elektroimpedanztomographie-Gerät und ein entsprechendes Verfahren anzugeben, mit denen die lokale Alveolardruck-Volumen-Hysterese und somit die dissipierte alveolare Atemarbeit bzw. eine dazu proportionale Größe lokal erfasst werden kann und diese ortsaufgelöst, visuell einfach erfassbar dargestellt werden kann.

Zur Lösung dieser Aufgabe dient ein Elektroimpedanzthomografie-Gerät mit den Merkmalen des Patentanspruchs 1 und ein Verfahren zur Aufnahme einer Folge von EIT-Bildern mit den Merkmalen des Patentanspruchs 13.

Erfindungsgemäß ist vorgesehen, dass die Steuer- und Auswerteeinheit dazu eingerichtet ist, die Zeitreihe der Matrizen auszuwerten, um für jedes Bildelement oder eine ausgewählte Menge von Bildelementen (z.B. eines ausgewählten Bildbereichs) jeweils die lokale Impedanzänderung über die mindestens eine Inspiration und die mindestens eine Exspiration als Funktion einer vom mittleren alveolaren Druck streng monoton abhängigen Größe als lokale Inspirationskurve und lokale Exspirationskurve zu erfassen. Die Größe, gegen diese Inspirations- und Exspirationskurven als aufgetragen betrachtet werden, kann der mittlere alveolare Druck selbst, eine lineare Funktion davon oder eben eine davon streng monoton abhängige Funktion sein, wobei letzteres impliziert, dass jede Änderung des mittleren alveolaren Drucks eine Änderung der betrachteten Größe in eindeutiger Richtung zur Folge hat. Anders ausgedrückt gibt es eine umkehrbar eindeutige Abbildung des mittleren alveolaren Drucks auf die davon streng monoton abhängige Größe; dies gilt natürlich insbesondere für eine linear abhängige Größe.

Die Zeitreihe der lokalen Impedanzänderungen besteht zwar aus diskreten Wertepaaren in dem von den genannten Größen aufgespannten Koordinatensystem, diese können aber stückweise durch gerade Strecken oder durch Anpassung von vorgegebenen Funktionen zu einer Kurve verbunden werden. Es werden für die betrachteten Bildelemente (dies können alle Bildelemente oder auch nur Bildelemente aus ausgewählten Bildbereichen sein) die Impedanzänderungskurven als lokale Inspirations- und Exspirationskurven erfasst.

Die lokalen Impedanzänderungskurven sind im Allgemeinen geschlossen, da die Lunge nach einer Inspiration und einer Exspiration wieder in den gleichen Druck- und Volumenzustand zurückkehrt. Die lokalen Impedanzänderungskurven verlaufen in einem Diagramm, dessen eine Koordinatenachse dem Volumen entspricht und dessen andere streng monoton zum Druck ist, insbesondere proportional dazu ist, so dass eine Art von P-V-Diagramm jedes Bildelements gegeben ist. Im Idealfall eines vollkommenen elastischen Lungenbereichs fallen Inspirations- und Exspirationskurventeil der Impedanzänderungskurve als Geraden zusammen. Im allgemeinen Fall sorgen aber nicht-elastische Verluste dafür, dass der Inspirationskurventeil und der Exspirationskurventeil nicht zusammenfallen, sondern eine Hysteresefläche umschließen. Die Steuer- und Auswerteeinheit ist weiter dazu eingerichtet, für jeweils einen die Abweichung zwischen lokalen Inspirations- und Exspirationskurve repräsentierenden Parameter als lokalen Dissipationswert des Bildelements zu berechnen. Die Steuer- und Auswerteeinheit ist weiter dazu eingerichtet, jeweils Bildelementen ihre lokalen Dissipationswerte oder daraus durch Normierung gewonnene Dissipationswerte zuzuordnen, die Dissipationswerte zu einer Dissipationskarte zusammenzustellen, in der den Bildelementen von ihren lokalen Dissipationswerten abhängige graphische Kodierungen zugewiesen sind, und die Dissipationskarte in der durch die Elektrodenebene definierten Bildebene auf der Anzeigeeinrichtung zur Anzeige zu bringen.

In einer bevorzugten Ausführungsform werden die Inspirations- und Exspirationskurven jedes betrachteten Bildelements als geschlossene Kurven erfasst, die im nichtelastischen Fall (analog zu geschlossen Kurven bei Magnetisierungen) als Hystereseflächen bezeichnet werden. In dieser Ausführungsform ist die Steuer- und Auswerteeinheit weiter dazu eingerichtet, als den die Abweichung zwischen Inspirations- und Exspirationskurve repräsentierenden Parameter die zwischen dem Inspirations- und Exspirationskurventeil liegende Fläche als Hysteresefläche des Bildelements zu berechnen, jedem betrachteten Bildelement seine Hysteresefläche als Dissipationswert oder einen daraus durch Normierung gewonnenen Dissipationswert zuzuordnen und diese zu einer Dissipationskarte zusammenzustellen, in der jedem Bildelement eine von seinem Hysteresewert abhängige graphische Kodierung zugewiesen ist, und die Hysteresekarte der Bildebene auf der Anzeigeeinrichtung zur Anzeige zu bringen.

Ein Vorteil der Anzeige einer Dissipationskarte liegt in der schnellen Erfassbarkeit von regionalen alveolaren Atemarbeitsverlusten während eines vollständigen Atemzugs, da sowohl die lokalen Inspirations- als auch Exspirationskurventeile der lokalen Impedanzänderungskurven verwendet werden. Da die mechanische Energie wie bei gesunden Alveolen in der Regel elastisch gespeichert wird, deuten alveolare Atemarbeitsverluste auf nicht-elastische Störungen während der Atmung hin, was bei kranken Lungen und/oder bei nicht optimal eingestellten Parametern eines Beatmungsgerätes auftreten kann.

Als ein anderes Beispiel für einen die Abweichung zwischen Inspirations- und Exspirationskurve repräsentierenden Parameter könnte z.B. der Betrag der Differenz der Integrale der Inspirations- und der Exspirationskurve über den beiden gemeinsamen Argumentbereich verwendet werden; diese Fläche zischen den Kurven liefert auch bei nicht geschlossenen Kurven einen die Abweichung charakterisierenden Parameter und entspricht im Fall geschlossener Kurven der Hysteresefläche.

In einer vorteilhaften Ausführungsform ist vorgesehen, dass die lokalen Impedanzänderungskurven aller betrachteten Bildelemente des Lungenquerschnitts mit einem gemeinsamen Faktor so normiert werden, dass die Summe aller normierten lokalen Impedanzänderungskurven gleich einer unabhängig bestimmten Volumenkurve der Respiration ist. Dadurch sind die normierten lokalen Impedanzänderungskurven als Volumenkurven zu interpretieren. Die unabhängig bestimmte Volumenkurve der Respiration kann zum Beispiel durch ein Beatmungsgerät bereitgestellt werden, das eine künstliche Beatmung des Patienten durchführt.

Die von der Steuer- und Auswerteeinheit verwendete, vom mittleren alveolaren Druck streng monoton abhängige Größe kann von der Steuer- und Auswerteeinheit auf verschiedene Weise bestimmt werden. In einer bevorzugten Ausführungsform empfängt die Steuer- und Auswerteeinheit unabhängig bestimmte Druck- und Volumenkurven der Respiration und bestimmt daraus mittels eines atemmechanischen Modells den mittleren alveolaren Druck und verwendet den mittleren alveolaren Druck selbst als die genannte Größe. Dies kann zum Beispiel durch quadratische Minimierung bezüglich der Parameter R und C aus obiger Bewegungsgleichung erfolgen, wobei die Druck- und Volumenkurven der Respiration po(t) und V(t) aus einem den Patienten künstlich beatmenden Beatmungsgerät stammen. In diesem Fall ist die vom mittleren alveolaren Druck streng monoton abhängige Größe eine davon linear abhängige Größe.

Alternativ kann die Steuer- und Auswerteeinheit dazu eingerichtet sein, als eine von dem mittleren alveolaren Druck lienar abhängige (und damit auch streng monoton abhängige) Größe eine unabhängig bestimmte Volumenkurve der Respiration zu verwenden, die bei angenommener elastischer Verformung eine lineare Funktion des mittleren alveolaren Druckes ist. Die unabhängig bestimmte Volumenkurve kann wiederum aus einem Beatmungsgerät stammen.

In einer weiteren alternativen Ausführungsform kann die Steuer- und Auswerteeinheit weiter dazu eingerichtet sein, als die von dem mittleren alveolaren Druck streng monoton abhängige Größe eine davon linear abhängige Größe in Form der globalen Impedanzänderungskurve, die aus der Summe aller lokalen Impedanzänderungskurven bestimmt wird, zu verwenden, die der Volumenkurve der Respiration und somit der Kurve des mittleren alveolaren Drucks bei angenommener elastischer Verformung proportional ist.

In einer bevorzugten Ausführungsform kann die Steuer- und Auswerteeinheit dazu eingerichtet sein, die von dem mittleren alveolaren Druck streng monoton abhängige Größe so zu normieren, dass diese am Ende der Exspiration gleich dem endexspiratorischen Druck (PEEP) und am Ende der Inspiration gleich dem maximalen Inspirationsdruck (PMAX) ist, so dass die lokalen Impedanzänderungskurven als p-V-Kurven gegeben sind.

In einer alternativen Ausführungsform kann die Steuer- und Auswerteeinheit dazu eingerichtet sein, die Hystereseflächen zu normieren, indem jede Hysteresefläche durch das Produkt der Differenz zwischen den endrespiratorischen Werten der lokalen Impedanzkurve und der Differenz zwischen den endrespiratorischen Werten der dem mittleren alveolaren Druck proportionalen Größe zu dividieren, so dass sich normierte Hystereseflächen zwischen 0 (keine Hysterese) und 1 (maximale Hysterese) ergeben. Anschaulich bedeutet dies, dass um die Hysteresekurve das kleinste die Hysteresekurve einschließende Rechteck (mit den Recheckseiten parallel zu den Koordinatenachsen) gelegt wird und die von der Hysteresekurve eingenommene Fläche durch die Fläche des darumgelegten Rechtecks geteilt wird. Im ideal elastischen Fall ist die Fläche der Hysteresekurve 0 und somit der normierte Hysteresewert 0. Im entgegengesetzten extremen Grenzfall folgt die Hysteresekurve dem geraden Verlauf des Rechtecks, so dass Hysteresefläche und Rechteckfläche gleich sind und sich ein extremer Hysteresewert 1 ergibt.

In einer bevorzugten Ausführungsform kann die Steuer- und Auswerteeinheit dazu eingerichtet sein, jeder, gegebenenfalls normierten, Hysteresefläche ein Vorzeichen zuzuordnen, indem geprüft wird, ob der Exspirationskurventeil der lokalen Impedanzänderungskurve als Funktion der dem mittleren Alveolardruck proportionalen Größe oberhalb des Inspirationskurventeils liegt, und wenn ja, das Vorzeichen + zuzuordnen, und wenn nein, das Vorzeichen - zuzuordnen. Die Vorzeichenzuordnung kann auch umgekehrt erfolgen.

Die Steuer- und Auswerteeinheit kann dazu eingerichtet sein, die Dissipationskarte mit der graphischen Kodierung der Hysteresewerte in Form einer Farb- oder Grauwertskala auf der Anzeigeeinrichtung zur Anzeige zu bringen.

Die Steuer- und Auswerteeinheit kann dazu eingerichtet sein, eine gemittelte Dissipationskarte zu bilden, indem entweder zu jedem betrachteten Bildelement die lokalen Impedanzänderungskurven über mehrere Atemzüge erfasst und gemittelt werden und die Dissipationskarte auf Grundlage der gemittelten lokalen Impedanzänderungskurven bestimmt wird oder die Dissipationskarten mehrerer Atemzüge gemittelt werden, und die gemittelte Dissipationskarte zur Anzeige zu bringen.

In einer vorteilhaften Ausführungsform kann die Steuer- und Auswerteeinheit dazu eingerichtet sein, einer Dissipationskarte durch Mittelwertbildung der Werte oder ihrer Beträge, Aufsummierung dieser oder durch Bestimmung des Maximalwertes aller Werte oder Beträge der Dissipationskarte einen globalen alveolaren Dissipationswert zuzuordnen und diesen numerisch oder graphisch auf der Anzeigeeinrichtung zur Anzeige zu bringen.

Die Erfindung beinhaltet ferner ein Verfahren entsprechend Anspruch 13.

Die im Zusammenhang mit der Erfindung betrachtete mindestens eine Inspiration und die mindestens eine Exspiration, die aufeinander folgen, sind typischwerweise ein Atemzug mit einer Inspiration und einer darauf folgenden Exspiration. Es ist jedoch grundsätzlich auch möglich, zunächst mit einer Exspiration zu beginnen und die darauf folgende Inspiration dazu zu betrachten; grundsätzlich können auch noch weitere Inspirationen und Exspirationen in die Analyse einbezogen werden.

Die Erfindung wird im Folgenden anhand eines Ausführungsbeispiels erläutert, wobei in

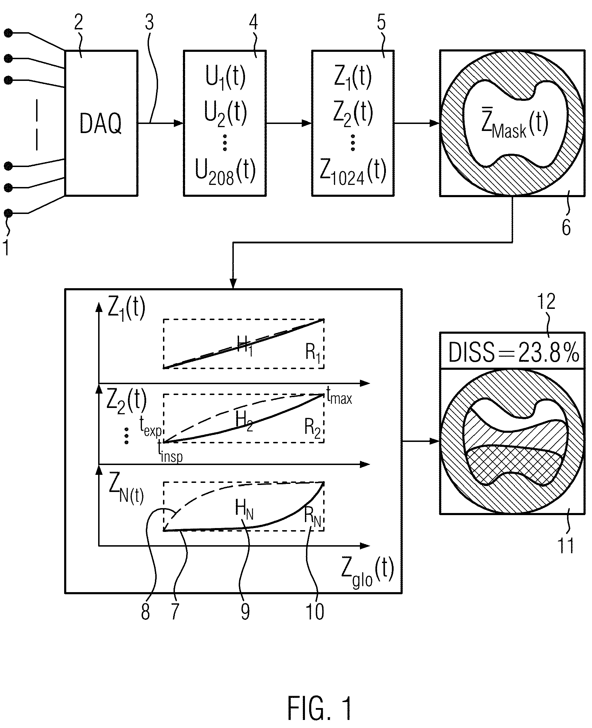

Fig. 1 ein schematisches Blockschaltbild eines Elektroimpedanztomografie-Gerätes gezeigt ist.

Bei dem in Fig. 1 schematisch als Blockschaltbild dargestellten EIT-Gerät wird zum Beispiel ein 16 Elektroden-System verwendet, wobei die Elektroden 1 ringförmig um den Thorax angebracht werden können. Über die Datenakquisitionseinheit 2 des EIT-Gerätes erfolgen Stromeinspeisung über ein Paar (vorzugsweise)benachbarter Elektroden, Spannungsmessungen über mehrere der anderen benachbarten Elektrodenpaare, dann Stromeinspeisung über ein anderes Paar benachbarter Elektroden, etc. bis mehrere oder alle benachbarten Elektrodenpaare einmal einspeisendes Elektrodenpaar waren; die dabei erhaltenen Messspannungen werden auch als Frame bezeichnet. Ein Frame von Messspannungen besteht hier aus 208 Spannungswerten, die mit Frame-Raten zwischen 10 Hz und 50 Hz aufgenommen werden können. Die Anzahl von 208 Messspannungen ergibt sich bei einem 16 Elektrodensystem dann, wenn jedes der 16 Paare benachbarter Elektroden einmal als einspeisendes Elektrodenpaar verwendet wird, wobei es jeweils unter den verbleibenden 14 Elektroden 13 unterschiedliche Paare benachbarter Elektroden gibt, zwischen denen eine Messspannung erfasst wird, so dass insgesamt bei einem Durchlauf 16x13 = 208 Messspannungen erfasst werden. Dies ist eine typische Ansteuerungsweise für ein EIT-Gerät mit einem 16 Elektrodensystem. Es ist aber in technischen Umsetzungen von EIT-Geräten auch vorstellbar, dass nicht alle der vorhandenen Elektroden zur Einspeisung von Strom oder Spannung genutzt werden, sondern einzelne Elektroden oder Elektrodenpaare bei der Einspeisung übersprungen werden. Ebenso ist es vorstellbar, dass Spannungsmessungen oder Strommessungen nicht an allen der vorhandenen Elektroden vorgenommen werden, sondern einzelne Elektroden oder Elektrodenpaare übersprungen und bei den Messungen ausgelassen werden.

Im vorliegenden Ausführungsbeispiel werden die 208 Zeitreihen der Spannungen $u_1(t)$, $u_2(t)$, ... $u_{208}(t)$ werden über ein Bussystem 3 zur Speichereinheit 4 des EIT-Geräts oder einer externen Speichereinheit geleitet. Aus den gemessenen Spannungen werden 1024 (relative) Impedanzänderungen $Z_1(t)$, $Z_2(t)$, ... $Z_{1024}(t)$ rekonstruiert, die auf ein Bild mit 32 x 32 Bildelementen abgebildet werden. Die Rekonstruktion und weitere Analysen können sowohl online als auch zeitlich und räumlich versetzt offline erfolgen. Die im Folgenden entstehenden Daten können daher intern im EIT-System über ein Bussystem oder in einem externen Speicher abgelegt werden. Es im Folgenden nicht mehr zwischen beiden Möglichkeiten unterschieden.

Ein Rechenmodul rekonstruiert aus den 208 Zeitreihen der Spannungen $u_1(t)$, $u_2(t)$, ... $u_{208}(t)$ die 1024 Zeitreihen der relativen Impedanzänderungen $Z_1(t)$, $Z_2(t)$, ... $Z_{1024}(t)$ und stellt sie in einen lokalen Impedanzmodul 5 bereit. Mit einer vorgegebenen oder aus den Daten bestimmten Bildelement-Maske 9 werden nur die informationstragenden "ventilierten" Bildelemente (Pixel) i = [1, 2, ... N] ausgewählt, d.h. die in Fig. 1 angedeuteten Randbereiche 6 außerhalb des werden ausgeblendet.

Durch Aufsummieren der lokalen Impedanzänderungen über alle informationstragenden Bildelemente wird eine Gesamtimpedanzänderung oder globale Impedanzänderung $Z_{glo}(t)$ bestimmt. Diese globale Impedanzänderungskurve $Z_{glo}(t)$ ist proportional zum respirierten Volumen und damit auch eine vom mittleren alveolaren Druck linear abhängige, und damit auch streng monoton abhängige, Größe.

$Z_{glo}(t)$ wird hier als die den mittleren alveolaren Druck repräsentierende (davon streng monoton abhängige) Größe verwendet. In Fig. 1 sind beispielhaft die lokalen Impedanzänderungskurven von drei Bildelementen als Funktion von $Z_{glo}(t)$ gezeigt. Dabei ist der Inspirationskurve 7 als durchgezogene Linie und der Exspirationskurve 8 als gestrichelte Linie gezeigt. Zwischen dieser Inspirationskurve 7 und der Exspirationskurve 8 liegt die Hystereseﬂäche 9, die hier für jedes Bildelement normiert wird, indem das kleinste die Inspirations- und Exspirationskurven 7 und 8 vollständig einschließende Rechteck 10 ermittelt wird und die Hystereseﬂäche $H_i$ des i-ten Bildelements dann durch die Fläche $R_i$ des kleinsten umschreibenden Rechtecks geteilt wird.

Die Verwendung der globalen Impedanzänderungskurve als die den mittleren alveolaren Druck repräsentierende Größe und die Normierung der Hystereseﬂächen durch die einschließende Rechteckfläche zur Berechnung der Dissipationswerte hat den Vorteil, dass so eine Dissipationskarte auch ohne Kenntnis von unabhängig bestimmten Druck- und Volumenkurven, z.B. aus einem Beatmungsgerät, bestimmt werden können. Da das Ergebnis der jeweiligen Dissipationswerte eine relative, auf sich selbst bezogene Größe ist, werden in dieser Definition Gebiete mit geringerem Ventilationsanteil durch die Normierung gleichwertig mit Gebieten mit hohem Ventilationsanteil behandelt, da gerade alveolare Lungengebiete mit schlechter Ventilation im Krankheitsfall in-elastisch arbeiten können.

Die globale Impedanzänderungskurve $Z_{glo}(t)$ des Atemzuges wird durch Aufsummierung aller N lokalen Impedanzänderungskurven $Z_i(t)$ innerhalb der Pixelmaske erzeugt, wobei N die Anzahl der betrachteten Bildelemente innerhalb der Pixelmaske ist:

$$Z_{glo}(t) = \frac{1}{N} \sum_{i=1}^{N} Z_i(t)$$

Um einen Bias zu vermeiden, kann das gerade analysierte Bildelement auch jeweils aus der Mittelwertbildung entfernt werden. Es wird nun die Abhängigkeit der lokalen Impedanzänderungskurve $Z_i(t)$ des aktuell analysierten Bildelements i von der globalen Impedanzänderungskurve $Z_{glo}(t)$ für die Inspirationsphase zwischen Beginn der Inspiration $t_{insp}$ und deren Ende bei $t_{max}$ als Kurve $2_i^{insp}(Z_{glo}(t))$ und für die Exspirationsphase zwischen Beginn bei $t_{max}$ und Ende bei $t_{exp}$ als Kurve $2_i^{esp}(Z_{glo}(t)$ für alle betrachteten Bildelemente i innerhalb der Pixelmaske betrachtet.

Die vorzeichenbehaftete Hystereseﬂäche $H_i$ für jedes Bildelement i ergibt sich zu

$$H_i = \int_{Z_{glo}(t_{exp})}^{Z_{glo}(t_{max})} Z_i^{exp}(Z_{glo}) dZ_{glo} - \int_{Z_{glo}(t_{insp})}^{Z_{glo}(t_{max})} Z_i^{insp}(Z_{glo}) dZ_{glo} \qquad \forall i \in \{1, 2..., N\}$$

Die kleinste umschließende Rechteckfläche für jedes Bildelement i ergibt sich zu:

$$R_i = (\max(Z_i) - \min(Z_i)) \cdot (\max(Z_{glo}) - \min(Z_{glo})) \qquad \forall i \in \{1, 2..., N\}$$

Als Dissipationswert des Bildelements i der Dissipationskarte wird die normierte Hystereseﬂäche

$$D_i = \frac{H_i}{R_i} \cdot 100\% \qquad\qquad \forall i \in \{1, 2 ..., N\}$$

definiert. Die Dissipationswerte liegen dann im Bereich von - 100% bis +100% und können auf der Anzeigeeinrichtung 11 farbkodiert dargestellt werden.

Bei betragsmäßig kleinen $D_i$-Werten um 0% liegt kaum Hystereseverhalten vor und die Alveolen arbeiten im elastischen Bereich, wie etwa in den Graphen $Z_1(t)$ in Fig. 1 gezeigt. $D_i$-Werte von zum Beispiel über 50%, wie in dem unteren der drei Graphen für $Z_N(t)$ in Fig. 1 gezeigt, deuten auf einen hohen Anteil von dissipativen Arbeitsverlusten durch nicht elastische Effekte hin. Für größere negative $D_i$-Werte ist die Interpretation nicht so klar. Meist gehören Bildelemente mit solchen Werten zur Klasse der nicht-ventilierten Bildelemente und könnten auf Flüssigkeitsverschiebung hindeuten.

Aus den lokalen Dissipationswerten der einzelnen Bildelemente wird durch Mittelwertbildung über die Dissipationskarte ein globaler Dissipationswert als skalare Größe definiert:

$$DISS = \frac{1}{N} \sum_{i=1}^{N} D_i$$

Der so definierte mittlere oder globale Dissipationswert kann auf der Anzeigeeinrichtung 11 in einem separaten Feld 12 als Zahlenwert angezeigt werden.

**Patentansprüche**

1.  Elektroimpedanztomographie-Gerät mit: einer Mehrzahl von um den Thorax eines Patienten anbringbaren, eine Elektrodenebene aufspannenden Elektroden (1), einer Anzeigeeinrichtung (11) und einer Steuer- und Auswerteeinheit, die mit den Elektroden verbunden ist und durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare aus der Mehrzahl der Elektroden ein Spannungssignal oder Stromsignal als Messsignale aufzunehmen, nacheinander andere Elektrodenpaare aus der Mehrzahl von Elektrodenpaaren als einspeisende Elektrodenpaare fungieren zu lassen, um aus den Messsignalen mit einem Rekonstruktionsalgorithmus eine Matrix aus Bildelementen zu rekonstruieren, die eine räumliche Verteilung von Impedanzänderungen in der Elektrodenebene repräsentiert, und über mindestens eine Inspiration und mindestens eine Exspiration, die aufeinander folgen, wiederholt Messsignale aufzunehmen und Matrizen zu rekonstruieren, um eine Zeitreihe von Matrizen mit Impedanzänderungen der Bildelemente über die mindestens eine Inspiration und die mindestens eine Exspiration zu erhalten, **dadurch gekennzeichnet, dass** die Steuer- und Auswerteeinheit weiter dazu eingerichtet ist, aus der Zeitreihe der Matrizen für jedes Bildelement oder eine ausgewählte Menge betrachteter Bildelemente jeweils die lokale Impedanzänderung des Bildelements als Funktion einer vom mittleren alveolaren Druck streng monoton abhängigen Größe über die mindestens eine Inspiration als lokale Inspirationskurve und über die mindestens eine Exspiration als lokale Exspirationskurve zu erfassen, jeweils einen die Abweichung zwischen lokaler Inspirations- und Exspirationskurve repräsentierenden Parameter als lokalen Dissipationswert des Bildelements zu berechnen, jeweils Bildelementen ihre lokalen Dissipationswerte oder daraus durch Normierung gewonnene lokale Dissipationswerte zuzuordnen, die lokalen Dissipationswerte aller betrachteten Bildelemente zu einer Dissipationskarte in einer durch die Elektrodenebene definierten Bildebene zusammenzustellen, in der den Bildelementen von ihren lokalen Dissipationswerten abhängige graphische Kodierungen zugewiesen sind, und die Dissipationskarte der Bildebene auf der Anzeigeeinrichtung zur Anzeige zu bringen.

2.  Elektroimpedanztomographie-Gerät nach Anspruch 1, **dadurch gekennzeichnet, dass** die Steuer- und Auswerteeinheit weiter dazu eingerichtet ist, die Inspirations- und Exspirationskurve jedes betrachteten Bildelements als geschlossene Kurven zu erfassen und die von ihnen eingeschlossene Hysteresefläche als Dissipationswert des Bildelements zu berechnen.

3.  Elektroimpedanztomographie-Gerät nach Anspruch 1 oder 2, **dadurch gekennzeichnet, dass** die Steuer- und Auswerteeinheit weiter dazu eingerichtet ist, die lokalen Impedanzänderungskurven aller Bildelemente mit einem gemeinsamen Faktor so zu normieren, dass die Summe aller normierten lokalen Impedanzänderungskurven gleich einer unabhängig bestimmten Volumenkurve der Respiration ist.

**4.** Elektroimpedanztomographie-Gerät nach Anspruch 1 oder 2, **dadurch gekennzeichnet, dass** die Steuer- und Auswerteeinheit weiter dazu eingerichtet ist, aus unabhängig bestimmten Druck- und Volumenkurven der Respiration den mittleren alveolaren Druck mittels eines atemmechanischen Modells zu bestimmen und diesen als die vom mittleren alveolaren Druck streng monoton abhängige Größe zu verwenden.

**5.** Elektroimpedanztomographie-Gerät nach Anspruch 1 oder 2, **dadurch gekennzeichnet, dass** die Steuer- und Auswerteeinheit weiter dazu eingerichtet ist, als die vom mittleren alveolaren Druck streng monoton abhängige Größe eine davon linear abhängige Größe in Form einer unabhängig bestimmte Volumenkurve der Respiration zu verwenden, die bei angenommener elastischer Verformung eine lineare Funktion des mittleren alveolaren Druckes ist.

**6.** Elektroimpedanztomographie-Gerät nach Anspruch 1 oder 2, **dadurch gekennzeichnet, dass** die Steuer- und Auswerteeinheit weiter dazu eingerichtet ist, als die von dem mittleren alveolaren Druck streng monoton abhängige Größe eine davon linear abhängige Größe in Form der aus der Summe aller lokalen Impedanzänderungskurven bestimmten globale Impedanzänderungskurve zu verwenden, die der Volumenkurve der Respiration und somit der Kurve des mittleren alveolaren Druckes bei angenommener elastischer Verformung proportional ist.

**7.** Elektroimpedanztomographie-Gerät nach Anspruch 1 oder 2, **dadurch gekennzeichnet, dass** die Steuer- und Auswerteeinheit weiter dazu eingerichtet ist, die vom mittleren alveolaren Druck streng monoton abhängige Größe so zu normieren, dass diese am Ende der Exspiration gleich dem endexspiratorischen Druck (PEEP) und am Ende der Inspiration gleich dem maximalen Inspirationsdruck (PMAX) ist, so dass die lokalen Impedanzänderungskurven p-V-Kurven darstellen.

**8.** Elektroimpedanztomographie-Gerät nach einem der Ansprüche 2 oder 2 bis 6, wenn abhängig von Anspruch 2, **dadurch gekennzeichnet, dass** die Steuer- und Auswerteeinheit weiter dazu eingerichtet ist, die Hystereseflächen zu Hysteresewerten zu normieren, indem jede Hysteresefläche durch das Produkt der Differenz zwischen den endrespiratorischen Werten der lokalen Impedanzkurve und Differenz zwischen den endrespiratorischen Werten der dem mittleren alveolaren Druck proportionalen Größe dividiert wird, so dass sich normierte Hystereseflächen zwischen 0 (keine Hysterese) und 1 (maximale Hysterese) ergeben.

**9.** Elektroimpedanztomographie-Gerät nach einem der Ansprüche 2 oder 3 bis 8, wenn abhängig von Anspruch 2, **dadurch gekennzeichnet, dass** die Steuer- und Auswerteeinheit weiter dazu eingerichtet ist, jeder, gegebenenfalls normierten, Hysteresefläche ein Vorzeichen zuzuordnen, indem geprüft wird, ob der Exspirationskurventeil der lokalen Impedanzänderungskurve als Funktion der dem mittleren Alveolardruck proportionalen Größe oberhalb des Inspirationskurventeils liegt, und wenn ja, ein Vorzeichen von + und - zugeordnet wird, und wenn nein, das umgekehrte Vorzeichen zugeordnet wird.

**10.** Elektroimpedanztomographie-Gerät nach einem der vorhergehenden Ansprüche, **dadurch gekennzeichnet, dass** die Steuer- und Auswerteeinheit weiter dazu eingerichtet ist, die Dissipationskarte mit der graphischen Kodierung in Form einer Farb- oder Grauwertskala auf der Anzeigeeinrichtung zur Anzeige zu bringen.

**11.** Elektroimpedanztomographie-Gerät nach einem der vorhergehenden Ansprüche, **dadurch gekennzeichnet, dass** die Steuer- und Auswerteeinheit weiter dazu eingerichtet ist, eine gemittelte Dissipationskarte zu bilden, indem entweder zu jedem Bildelement die lokalen Impedanzänderungskurven über mehrere Atemzüge erfasst und gemittelt werden und die Dissipationskarte auf Grundlage der gemittelten lokalen Impedanzänderungskurven bestimmt wird oder die Dissipationskarten mehrerer Atemzüge gemittelt werden, und die gemittelte Dissipationskarte zur Anzeige zu bringen.

**12.** Elektroimpedanztomographie-Gerät nach einem der vorhergehenden Ansprüche, **dadurch gekennzeichnet, dass** die Steuer- und Auswerteeinheit weiter dazu eingerichtet ist, einer Dissipationskarte durch Mittelwertbildung, Aufsummierung oder durch Bestimmung des Maximalwertes aller Werte oder Beträge aus der Dissipationskarte einen globalen alveolaren Dissipationswert zuzuordnen und diesen numerisch oder graphisch auf der Anzeigeeinrichtung zur Anzeige zu bringen.

**13.** Verfahren zur Aufnahme einer Folge von EIT-Bildern einer Querschnittsebene des Thorax eines Patienten durch eine Mehrzahl von auf dem Umfang des Thorax verteilten, eine Elektrodenebene aufspannenden Elektroden (1), wobei bei dem Verfahren durch eine Steuer- und Auswerteeinheit ein Elektrodenpaar als einspeisendes Elektrodenpaar mit einem Wechselstrom oder einer Wechselspannung versorgt wird, mit mehreren der übrigen Elektrodenpaare Spannungssignale oder Stromsignale als Messsignale aufgenommen werden und sukzessive andere Elektrodenpaare aus der Mehrzahl von Elektrodenpaaren als einspeisende Elektrodenpaare betrieben werden, aus der Gesamtheit der Messsignale mit einem Rekonstruktionsalgorithmus eine Matrix aus Bildelementen rekonstruiert wird, die die räumliche Verteilung der Impedanzänderungen Bildelement für Bildelement in der Elektrodenebene repräsentiert, über die Zeit wiederholt Matrizen der Impedanzänderung rekonstruiert werden, um eine Zeitreihe von Matrizen der lokalen Impedanzänderungen über mindestens eine Inspiration und mindestens eine Exspiration, die aufeinander folgen, zu erhalten, **dadurch gekennzeichnet, dass** durch die Steuer- und Auswerteeinheit aus der Zeitreihe der Matrizen für jedes Bildelement oder eine ausgewählte Menge von Bildelementen jeweils die lokale Impedanzänderung als Funktion einer vom alveolaren Druck streng monoton abhängigen Größe über die mindestens eine Inspiration als lokale Inspirationskurve und die mindestens eine Exspiration als lokale Exspirationskurve erfasst und ein die Abweichung zwischen lokaler Inspirations- und Exspirationskurve repräsentierender Parameter als lokaler Dissipationswert des Bildelements berechnet wird, die lokalen Dissipationswerte aller betrachteten Bildelemente zu einer Dissipationskarte in einer durch die Elektrodenebene definierten Bildebene zusammengestellt werden, in der jedem betrachteten Bildelement eine von ihrem Dissipationswert abhängige graphische Kodierung zugewiesen ist, und die Dissipationskarte der Bildebene auf der Anzeigeeinrichtung (11) zur Anzeige gebracht wird.

## Claims

1. An electro-impedance tomography apparatus having: a plurality of electrodes (1) that span an electrode plane and can be fitted around the thorax of a patient, a display device (11) and a control and evaluation unit that is connected to the electrodes and is set up by programming to supply at least one pair of electrodes, as a feeding pair of electrodes, with an alternating current or with an alternating voltage, to receive with several of the remaining pairs of electrodes of the plurality of the electrodes a voltage signal or current signal as measurement signals, to allow other pairs of electrodes of the plurality of pairs of electrodes to function one after the other as feeding pairs of electrodes in order to reconstruct out of the measurement signals with a reconstruction algorithm a matrix of image elements that represents a spatial distribution of changes in impedance in the electrode plane, and by way of at least one inspiration and at least one expiration that follow each other to receive repeatedly measurement signals and to reconstruct matrices in order to obtain a time series of matrices with changes in impedance of the image elements by way of the at least one inspiration and the at least one expiration, **characterised in that** the control and evaluation unit is set up, furthermore, to detect from the time series of the matrices for each image element or a selected quantity of image elements considered respectively the local change in impedance of the image element as a function of a variable, which is strictly monotonically dependent on the average alveolar pressure, by way of the at least one inspiration as a local inspiration curve and by way of the at least one expiration as a local expiration curve, to calculate respectively a parameter representing the deviation between the local inspiration curve and expiration curve as a local dissipation value of the image element, to allocate respectively to image elements their local dissipation values or local dissipation values gained therefrom by standardization, to compile the local dissipation values of all the image elements considered in a dissipation map in an image plane which is defined by the electrode plane and in which there are assigned to the image elements graphic codings that are dependent on their local dissipation values, and to display the dissipation map of the image plane on the display device.

2. An electro-impedance tomography apparatus according to claim 1, **characterised in that** the control and evaluation unit is set up, furthermore, to detect the inspiration curve and expiration curve of each image element considered as closed curves and to calculate the hysteresis area enclosed by them as a dissipation value of the image element.

3. An electro-impedance tomography apparatus according to claim 1 or 2, **characterised in that** the control and evaluation unit is set up, furthermore, to standardize the local impedance-change curves of all the image elements with a common factor so that the sum of all the standardized local impedance-change curves is equal to an independently determined volume curve of the respiration.

4. An electro-impedance tomography apparatus according to claim 1 or 2, **characterised in that** the control and evaluation unit is set up, furthermore, to determine the average alveolar pressure from independently determined pressure and volume curves of the respiration by means of a model of a respiration mechanism and to use this as the variable that is strictly monotonically dependent on the average alveolar pressure.

5. An electro-impedance tomography apparatus according to claim 1 or 2, **characterised in that** the control and evaluation unit is set up, furthermore, to use as the variable that is strictly monotonically dependent on the average alveolar pressure a variable that is linearly dependent thereon in the form of an independently determined volume curve of the respiration which, given assumed elastic deformation, is a linear function of the average alveolar pressure.

6. An electro-impedance tomography apparatus according to claim 1 or 2, **characterised in that** the control and evaluation unit is set up, furthermore, to use as the variable that is strictly monotonically dependent on the average alveolar pressure a variable that is linearly dependent thereon in the form of the global impedance-change curve that is determined from the sum of all the local impedance-change curves and is proportional to the volume curve of the respiration and thus the curve of the average alveolar pressure, given assumed elastic deformation.

7. An electro-impedance tomography apparatus according to claim 1 or 2, **characterised in that** the control and evaluation unit is set up, furthermore, to standardize the variable that is strictly monotonically dependent on the average alveolar pressure in such a way that at the end of the expiration it is equal to the end expiratory pressure (PEEP) and at the end of the inspiration it is equal to the maximum inspiration pressure (PMAX) so that the local impedance-change curves represent p-v-curves.

8. An electro-impedance tomography apparatus according to one of claims 2 or 2 to 6, if dependent on claim 2, **characterised in that** the control and evaluation unit is set up, furthermore, to standardize the hysteresis areas at hysteresis values by dividing each hysteresis area by the product of the difference between the end respiratory values of the local impedance curve and the difference between the end respiratory values of the variable that is proportional to the average alveolar pressure so that standardized hysteresis areas between 0 (no hysteresis) and 1 (maximum hysteresis) result.

9. An electro-impedance tomography apparatus according to one of claims 2 or 3 to 8, if dependent on claim 2, **characterised in that** the control and evaluation unit is set up, furthermore, to allocate a sign to each, if applicable standardized, hysteresis area by examining whether the expiration-curve portion of the local impedance-change curve as a function of the variable that is proportional to the average alveolar pressure lies above the inspiration-curve portion, and, if so, allocating a + and - sign and, if not, allocating the opposite sign.

10. An electro-impedance tomography apparatus according to one of the preceding claims, **characterised in that** the control and evaluation unit is set up, furthermore, to display the dissipation map with the graphic coding in the form of a colour-value or grey-value scale on the display device.

11. An electro-impedance tomography apparatus according to one of the preceding claims, **characterised in that** the control and evaluation unit is set up, furthermore, to establish an averaged dissipation map either by detecting and averaging the local impedance-change curves over several breaths for each image element and determining the dissipation map on the basis of the averaged local impedance-change curves or by averaging the dissipation maps of several breaths, and to display the averaged dissipation map.

12. An electro-impedance tomography apparatus according to one of the preceding claims, **characterised in that** the control and evaluation unit is set up, furthermore, to allocate a global alveolar dissipation value to a dissipation map by establishing an average value, summing up or by determining the maximum value of all the values or amounts from the dissipation map, and to display this value numerically or graphically on the display device.

13. A method for receiving a sequence of EIT images of a cross-sectional plane of the thorax of a patient by means of a plurality of electrodes (1) spanning an electrode plane and distributed over the periphery of the thorax,
wherein in the method by means of a control and evaluation unit a pair of electrodes, as a feeding pair of electrodes, is supplied with an alternating current or an alternating voltage, voltage signals or current signals as measurement signals are received with several of the remaining pairs of electrodes, and successively other pairs of electrodes of the plurality of pairs of electrodes are operated as feeding pairs of electrodes,
a matrix of image elements that represents the spatial distribution of the changes in impedance image element by image element in the electrode plane is reconstructed out of all of the measurement signals with a reconstruction algorithm,
over time repeatedly matrices of the change in impedance are reconstructed in order to obtain a time series of matrices of the local changes in impedance by way of at least one inspiration and at least one expiration that follow each other,
**characterised in that** by means of the control and evaluation unit there is detected from the time series of the matrices for each image element or a selected quantity of image elements respectively the local change in impedance as a function of a variable, which is strictly monotonically dependent on the alveolar pressure, by way of the at least one inspiration as a local inspiration curve and the at least one expiration as a local expiration curve, and a parameter representing the deviation between the local inspiration curve and expiration curve is calculated as a local dissipation value of the image element, the local dissipation values of all the image elements considered are compiled in a dissipation map in an image plane which is defined by the electrode plane and in which there is assigned to each image element considered a graphic coding that is dependent on their local dissipation value, and the dissipation map of the image plane is displayed on the display device (11).

**Revendications**

1. Appareil de tomographie d'impédance électrique comportant : une pluralité d'électrodes (1) pouvant être disposées autour du thorax d'un patient, définissant un plan d'électrodes, un dispositif d'affichage (11) et une unité de commande et d'évaluation qui est reliée aux électrodes et conçue par programmation pour alimenter au moins une paire d'électrodes, utilisée comme paire d'électrodes d'alimentation, avec un courant alternatif ou avec une tension alternative, acquérir avec plusieurs des paires d'électrodes restantes de la pluralité d'électrodes un signal de tension ou un signal de courant en tant que signaux de mesure, faire fonctionner successivement d'autres paires d'électrodes de la pluralité de paires d'électrodes comme paires d'électrodes d'alimentation, pour, à partir des signaux de mesure, reconstruire avec un algorithme de reconstruction une matrice d'éléments d'image qui représente une distribution spatiale de variations d'impédance dans le plan d'électrodes, et, sur au moins une inspiration et au moins une expiration qui se suivent, acquérir de manière répétée des signaux de mesure et reconstruire des matrices pour obtenir une série chronologique de matrices avec des variations d'impédance des éléments d'image sur ladite au moins une inspiration et ladite au moins une expiration, **caractérisé en ce que** l'unité de commande et d'évaluation est en outre conçue ou réalisée pour, à partir de la série chronologique des matrices, pour chaque élément d'image ou un ensemble sélectionné d'éléments d'image considérés, enregistrer chaque fois la variation locale d'impédance de l'élément d'image en fonction d'une grandeur dépendant d'une manière strictement monotone de la pression alvéolaire moyenne sur ladite au moins une inspiration en tant que courbe locale d'inspiration et sur ladite au moins une expiration en tant que courbe locale d'expiration, calculer chaque fois un paramètre représentant l'écart entre courbe locale d'inspiration et d'expiration en tant que valeur locale de dissipation de l'élément d'image, associer chaque fois aux éléments d'image leurs valeurs locales de dissipation ou des valeurs locales de dissipation obtenues à partir de celles-ci par normalisation, rassembler les valeurs locales de dissipation de tous les éléments d'image considérés en une carte de dissipation dans un plan d'image défini par le plan d'électrodes, dans laquelle des codages graphiques sont attribués aux éléments d'image en fonction de leurs valeurs locales de dissipation, et afficher la carte de dissipation du plan d'image sur le dispositif d'affichage.

2. Appareil de tomographie d'impédance électrique selon la revendication 1, **caractérisé en ce que** l'unité de commande et d'évaluation est en outre conçue ou réalisée pour enregistrer les courbes d'inspiration et d'expiration de chaque élément d'image considéré comme des courbes fermées et calculer la surface d'hystérésis qu'elles délimitent en tant que valeur de dissipation de l'élément d'image.

3. Appareil de tomographie d'impédance électrique selon la revendication 1 ou la revendication 2, **caractérisé en ce que** l'unité de commande et d'évaluation est en outre conçue ou réalisée pour normaliser les courbes locales d'impédance de tous les éléments d'image avec un facteur commun, de façon que la somme de toutes les courbes locales d'impédance normalisées soit égale à une courbe de volume de la respiration déterminée indépendamment.

4. Appareil de tomographie d'impédance électrique selon la revendication 1 ou la revendication 2, **caractérisé en ce que** l'unité de commande et d'évaluation est en outre conçue ou réalisée pour déterminer, à partir de courbes de pression et de volume de la respiration déterminées indépendamment, la pression alvéolaire moyenne au moyen d'un modèle de mécanique respiratoire et utiliser celle-ci comme grandeur dépendant d'une manière strictement monotone de la pression alvéolaire moyenne.

5. Appareil de tomographie d'impédance électrique selon la revendication 1 ou la revendication 2, **caractérisé en ce que** l'unité de commande et d'évaluation est en outre conçue ou réalisée pour utiliser comme grandeur dépendant d'une manière strictement monotone de la pression alvéolaire moyenne une grandeur qui en dépend linéairement sous la forme d'une courbe de volume de la respiration déterminée indépendamment, qui, en supposant une déformation élastique, est une fonction linéaire de la pression alvéolaire moyenne.

6. Appareil de tomographie d'impédance électrique selon la revendication 1 ou la revendication 2, **caractérisé en ce**

**que** l'unité de commande et d'évaluation est en outre conçue ou réalisée pour utiliser comme grandeur dépendant d'une manière strictement monotone de la pression alvéolaire moyenne une grandeur qui en dépend linéairement sous la forme de la courbe globale d'impédance déterminée à partir de la somme de toutes les courbes locales d'impédance, qui, en supposant une déformation élastique, est proportionnelle à la courbe de volume de la respiration et donc à la courbe de la pression alvéolaire moyenne.

7. Appareil de tomographie d'impédance électrique selon la revendication 1 ou la revendication 2, **caractérisé en ce que** l'unité de commande et d'évaluation est en outre conçue ou réalisée pour normaliser la grandeur dépendant d'une manière strictement monotone de la pression alvéolaire moyenne de façon que celle-ci soit égale à la pression expiratoire positive (PEEP) à la fin de l'expiration et à la pression inspiratoire maximale (PMAX) à la fin de l'inspiration, de sorte que les courbes locales d'impédance représentent des courbes p-V.

8. Appareil de tomographie d'impédance électrique selon l'une des revendications 2 ou 2 à 6 lorsqu' elle dépend de la revendication 2, **caractérisé en ce que** l'unité de commande et d'évaluation est en outre conçue ou réalisée pour normaliser les surfaces d'hystérésis à des valeurs d'hystérésis, en divisant chaque surface d'hystérésis par le produit de la différence entre les valeurs respiratoires finales de la courbe locale d'impédance et de la différence entre les valeurs respiratoires finales de la grandeur proportionnelle à la pression alvéolaire moyenne, de sorte qu'il en résulte des surfaces d'hystérésis normalisées comprises entre 0 (pas d'hystérésis) et 1 (hystérésis maximale).

9. Appareil de tomographie d'impédance électrique selon l'une des revendications 2 ou 3 à 8, lorsqu' elle dépend de la revendication 2, **caractérisé en ce que** l'unité de commande et d'évaluation est en outre conçue ou réalisée pour associer un signe à chaque surface d'hystérésis, le cas échéant normalisée, en contrôlant si la partie de courbe d'expiration de la courbe locale d'impédance en fonction de la grandeur proportionnelle à la pression alvéolaire moyenne se situe au-dessus de la partie de courbe d'inspiration, et si oui en lui associant un signe + et -, et si non en lui associant le signe opposé.

10. Appareil de tomographie d'impédance électrique selon l'une des revendications précédentes, **caractérisé en ce que** l'unité de commande et d'évaluation est en outre conçue ou réalisée pour afficher la carte de dissipation sur le dispositif d'affichage avec le codage graphique sous la forme d'une échelle de couleurs ou de gris.

11. Appareil de tomographie d'impédance électrique selon l'une des revendications précédentes, **caractérisé en ce que** l'unité de commande et d'évaluation est en outre conçue ou réalisée pour former une carte de dissipation moyenne soit en enregistrant et moyennant pour chaque élément d'image les courbes locales d'impédance sur plusieurs respirations et en déterminant la carte de dissipation sur la base des courbes locales d'impédance moyennées, soit en moyennant les cartes de dissipation de plusieurs respirations, et pour afficher la carte de dissipation moyenne.

12. Appareil de tomographie d'impédance électrique selon l'une des revendications précédentes, **caractérisé en ce que** l'unité de commande et d'évaluation est en outre conçue ou réalisée pour associer à une carte de dissipation, par formation de moyenne, sommation ou par détermination de la valeur maximale de toutes les valeurs ou montants de la carte de dissipation, une valeur de dissipation alvéolaire globale et pour afficher celle-ci numériquement ou graphiquement sur le dispositif d'affichage.

13. Procédé d'acquisition d'une suite d'images de tomographie d'impédance électrique d'un plan de coupe transversale du thorax d'un patient par une pluralité d'électrodes (1) réparties sur le pourtour du thorax, définissant un plan d'électrodes, le procédé consistant à

alimenter par une unité de commande et d'évaluation une paire d'électrodes utilisée comme paire d'électrodes d'alimentation avec un courant alternatif ou une tension alternative, acquérir des signaux de tension ou des signaux de courant en tant que signaux de mesure avec plusieurs des paires d'électrodes restantes et faire fonctionner successivement d'autres paires d'électrodes de la pluralité de paires d'électrodes comme paires d'électrodes d'alimentation,

reconstruire à partir de la totalité des signaux de mesure, avec un algorithme de reconstruction, une matrice d'éléments d'image qui représente la distribution spatiale des variations d'impédance élément d'image par élément d'image dans le plan d'électrodes,

reconstruire de manière répétée au cours du temps des matrices de variation d'impédance pour obtenir une série chronologique de matrices des variations locales d'impédance sur au moins une inspiration et au moins une expiration qui se suivent,

**caractérisé en ce que**, à partir de la série chronologique des matrices, pour chaque élément d'image ou un ensemble sélectionné d'éléments d'image, la variation locale d'impédance est enregistrée par l'unité de commande et d'évaluation en fonction d'une grandeur dépendant d'une manière strictement monotone de la pression alvéolaire sur ladite au moins une inspiration en tant que courbe locale d'inspiration et ladite au moins une expiration en tant que courbe locale d'expiration, et un paramètre représentant l'écart entre courbe locale d'inspiration et d'expiration est calculé en tant que valeur locale de dissipation de l'élément d'image, les valeurs locales de dissipation de tous les éléments d'image considérés sont rassemblées en une carte de dissipation dans un plan d'image défini par le plan d'électrodes, dans lequel un codage graphique est attribué à chaque élément d'image considéré en fonction de sa valeur de dissipation, et la carte de dissipation du plan d'image est affichée sur le dispositif d'affichage (11).

FIG. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

*   EP 1000580 A1

**In der Beschreibung aufgeführte Nicht-Patentliteratur**

*   A Unified Approach for EIT Imaging of Regional Overdistension and Atelectasis in Acute Lung Injury. **GOMEZ-LABERGE C et al.** IEEE TRANSACTIONS ON MEDICAL IMAGING. IEEE SERVICE CENTER, Marz 2012, vol. 31, 834-842

*   **G. KÜHNEL.** Neue Verfahren zur Verbesserung der Abbildungsqualität bei funktionellen EIT-Tomogrammen der Lunge. *Biomed. Tech.,* 1997, vol. 42, 470-1
*   Regional ventilation delay index: detection of tidal recruitment using electric impedance tomography. **T. MUDERS et al.** Yearbook of Intensive Care and Emergency Medicine